Europäisches Patentamt

European Patent Office

Office européen des brevets

(11) Veröffentlichungsnummer: **0 163 864**
**B1**

(12) **EUROPÄISCHE PATENTSCHRIFT**

(45) Veröffentlichungstag der Patentschrift:
**01.06.88**

(21) Anmeldenummer: **85104435.4**

(22) Anmeldetag: **12.04.85**

(51) Int. Cl.⁴: **B 29 C 43/48,** B 30 B 15/34,
B 27 N 3/24

(54) **Verfahren und Vorrichtung zum kontinuierlichen Verpressen von Werkstoffbahnen.**

(30) Priorität: **08.05.84 DE 3416985**

(43) Veröffentlichungstag der Anmeldung:
**11.12.85 Patentblatt 85/50**

(45) Bekanntmachung des Hinweises auf die Patenterteilung:
**01.06.88 Patentblatt 88/22**

(84) Benannte Vertragsstaaten:
**FR GB IT SE**

(56) Entgegenhaltungen:
**AT - B - 221 369**
**AT - B - 319 575**
**DE - A - 1 504 183**
**DE - A - 1 629 376**
**DE - A - 2 135 230**
**DE - A - 2 729 219**

(73) Patentinhaber: **Held, Kurt, Alte Strasse 1,
D-7218 Trossingen 2 (DE)**

(72) Erfinder: **Held, Kurt, Alte Strasse 1,
D-7218 Trossingen 2 (DE)**

**Beschreibung**

Die Erfindung betrifft ein Verfahren zum kontinuierlichen Verpressen von Werkstoffbahnen in einer Reaktionszone zwischen zwei endlosen, aufgeheizten Pressbändern einer Doppelbandpresse bei erhöhter Prozesstemperatur, wobei die Pressbänder so aufgeheizt werden, dass sie an ihren der Werkstoffbahn zugekehrten Aussenseiten die höchste Temperatur haben und der Wärmefluss durch das Pressband von dieser heissen Aussenseite zur gegenüberliegenden Innenseite des Pressbandes hin erfolgt. Ferner betrifft die Erfindung Vorrichtungen zur Durchführung eines solchen Verfahrens.

Bei bekannten Verfahren und Vorrichtungen dieser Art sind die Temperaturen, denen die zu verpressenden Werkstoffe, zum Beispiel kunststoffgebundene Laminate oder Faser-Bindemittelgemische, ausgesetzt werden können, begrenzt, und zwar in erster Linie durch die Wärme- und Oxidationsbeständigkeit der Schmierstoffe bei mechanisch, zum Beispiel durch Wälzkörper abgestützten Pressbändern oder durch die Wärmebeständigkeit der Dichtungswerkstoffe und des Druckmittels bei hydraulisch abgestützten Pressbändern. Es ist manchmal erwünscht, beispielsweise bei der Verdichtung von Faser-Bindemittelgemischen, wobei die Bindemittel Thermoplaste mit Schmelzpunkten zwischen etwa 250 und 350 °C sind, Pressbandtemperaturen zur Anwendung zu bringen, die um bis zu etwa 40 °C über den vorgenannten Temperaturwerten liegen. Herkömmliche Schmierstoffe und elastomere Dichtungsstoffe halten jedoch Betriebstemperaturen bis lediglich etwa 200 °C aus.

Bei den bekannten Doppelbandpressen wird die Wärme entweder durch Beheizen von Umlenktrommeln, auf denen die Pressbänder abgestützt sind, auf der Einlaufseite der Presse oder durch Beheizen einer Druckplatte, über welche die Pressbänder im Bereich der Reaktionszone abgestützt sind, oder auch durch ein heisses Druckmittel selbst auf die Innenseite der Pressbänder übertragen. Von der Innenseite der Pressbänder fliesst die Wärme durch die Pressbänder hindurch an deren Aussenseite und wird dort an die zwischen den Pressbändern liegende Werkstoffbahn abgegeben. Dabei herrschen naturgemäss an den Pressbandinnenseiten höhere Temperaturen als an den die Werkstoffbahn kontaktierenden Pressbandaussenseiten, was an sich ein technologischer Widerspruch ist, denn an der Pressbandinnenseite schadet die höhere Temperatur den verwendeten Dichtungen, Druckmitteln oder Schmierstoffen, während die (niedrigere) Temperaturen an den Pressbandaussenseiten häufig nicht ausreicht, um die erforderlichen Abbindereaktionen herbei zu führen.

Aus der AT-B-319 575 ist weiter ein Verfahren und eine Vorrichtung zur Herstellung von Schaumstoffkörpern aus vorgeschäumten und aus thermoplastischem Kunststoff bestehenden Perlen bekannt geworden, wobei die Perlen bis auf Erweichungstemperatur erhitzt, zu einem Formkörper zusammengepresst und dieser anschliessend unter Druck abgekühlt wird. Der Formkörperstrang wird zwischen zwei Transportbändern erwärmt, mit einer Oberflächenbeschichtung versehen und anschliessend zwischen zwei weiteren Transportbändern gepresst. Die Aussenseiten dieser Transportbänder können an den einlaufseitigen Umlenktrommeln mittels Infrarotstrahlern erwärmt werden.

Nachteilig ist bei diesem Verfahren, dass sofort nach Erwärmung der Aussenseiten der Transportbänder ein grosser Wärmefluss zur Innenseite dieser Transportbänder einsetzt. In der Regel bestehen solche Transportbänder aus Metall und setzen somit diesem Wärmefluss einen geringen Wärmewiderstand entgegen. Weiterhin sind die Infrarotstrahler auf einem weiten Teil des Umfangs der Umlenktrommeln angeordnet, so dass genügend Zeit für einen Wärmeausgleich zwischen Aussen- und Innenseiten der Transportbänder zur Verfügung steht und in die Innenseiten der Transportbänder damit beim Einlauf in die Presszone dieselbe Temperatur wie die Aussenseite besitzen. Um die zulässigen Betriebstemperaturen der Schmierstoffe und Dichtungswerkstoffe in der Presse nicht zu überschreiten, dürfen somit die Aussenseiten der Transportbänder ebenfalls nicht auf eine höhere als diese zulässige Betriebstemperatur erwärmt werden, womit dieses Verfahren zur Verpressung einer Werkstoffbahn bei erhöhter Prozesstemperatur in einer Doppelbandpresse nicht geeignet ist.

Ausgehend von diesem Stand der Technik ist es Aufgabe der Erfindung, ein Verfahren und Vorrichtungen vorzuschlagen, die das Behandeln von Werkstoffbahnen in Doppelbandpressen auch bei solchen Pressbandtemperaturen ermöglichen, welche höher als die zulässigen Betriebstemperaturen bestimmter Maschinenteile, wie Dichtungen, Gleitwerkstoffe, Druckmittel, Schmierstoffe, liegen.

Die Aufgabe wird bei dem erfindungsgemässen Verfahren dadurch gelöst, dass die Pressbänder aus mehreren, ineinander geschobenen Bandtrumen bestehen, alle Bandtrume von der Reaktionszone von ihrer Innenseite her vorgeheizt werden und lediglich die Aussenseite des aussen gelegenen Bandtrums kurz vor der Reaktionszone zusätzlich auf erhöhte Temperatur gebracht wird, so dass der Wärmefluss von der heissen Aussenseite des aussenliegenden Bandtrums zur gegenüberliegenden Innenseite des innenliegenden Bandtrums mit hohem Wärmewiderstand erfolgt. Geeignete Vorrichtungen zur Durchführung dieses Verfahrens umfassen erfindungsgemäss in der Nähe der Aussenseiten des aussenliegenden Bandtrums des aus mehreren, ineinander geschobenen Bandtrumen bestehenden Pressbandes kurz vor der Reaktionszone quer über die Breite der Pressbänder reichende, leistenförmige Gasbrenner oder elektrische Induktionsheizkörper mit von hochfrequenten Wechselströmen durchflossenen Koppelspulen.

Die nachstehende Beschreibung bevorzugter Ausführungsformen der Erfindung dient im Zusammenhang mit beiliegender Zeichnung der weiteren Erläuterung. Es zeigen:

Fig. 1 eine schematische Darstellung der Einlaufzone einer Doppelbandpresse;

Fig. 2 eine vergrösserte Schnittansicht entlang der Linie 2–2 in Fig. 1, und

Fig. 3 den Temperaturverlauf in einem Pressbandpaket vor und hinter einer Aufheizzone.

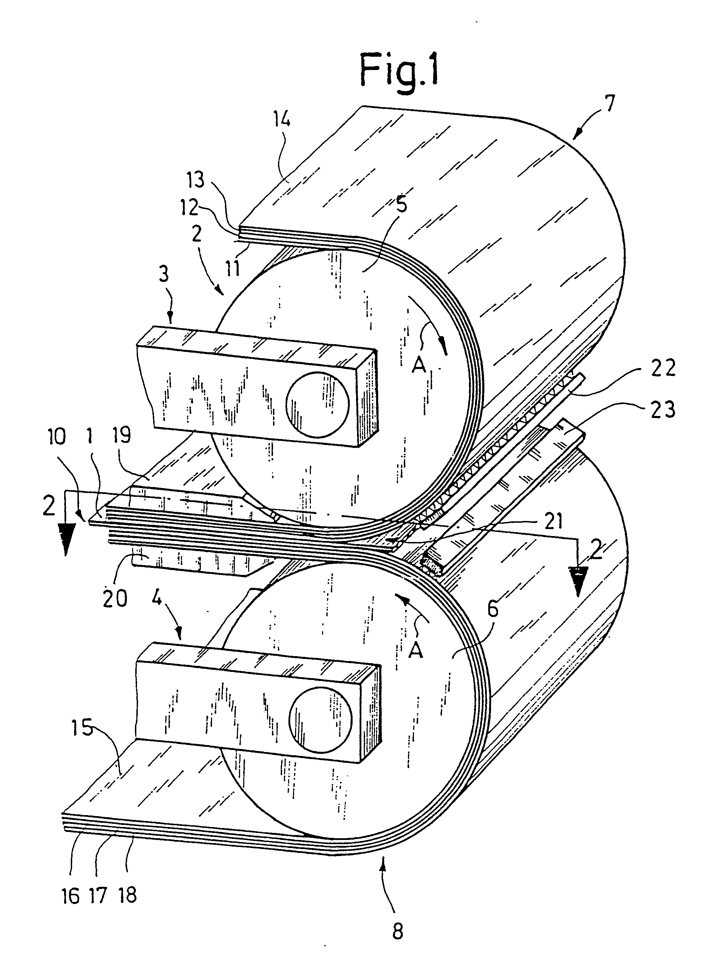

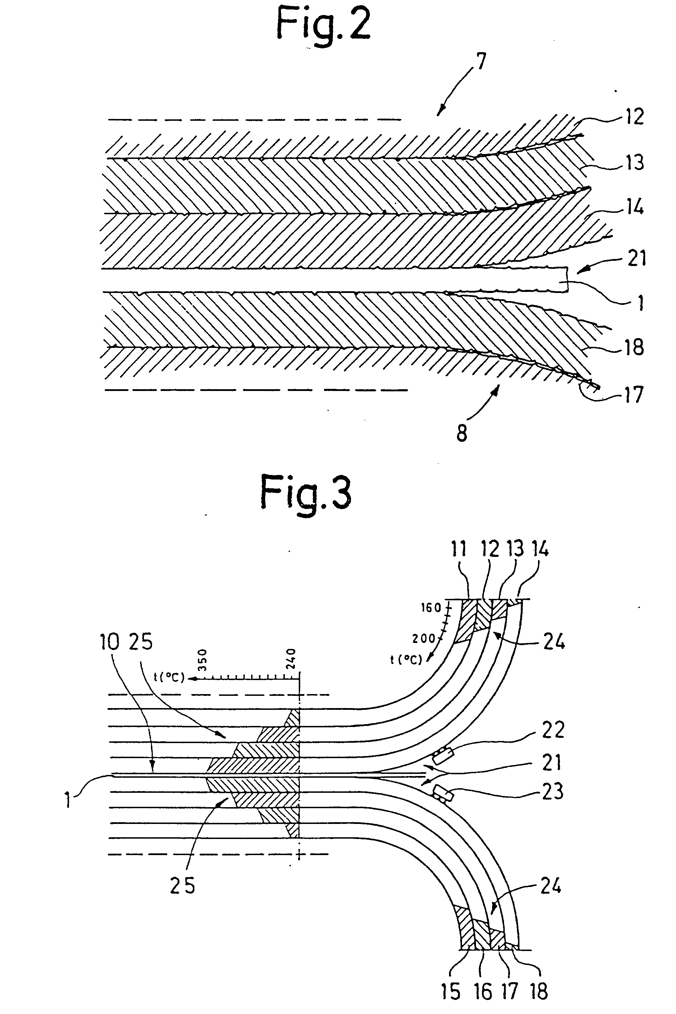

In Fig. 1 ist die Einlaufzone einer zum Heissverpressen einer vorlaufenden Werkstoffbahn 1 dienenden Doppelbandpresse 2 dargestellt. Bei der Werkstoffbahn 1 kann es sich um ein kunststoffgebundenes Laminat, zum Beispiel ein Elektrolaminat, oder auch um ein Faser-Bindemittelgemisch handeln, wobei das Bindemittel ein Thermoplast mit einem Schmelzpunkt zwischen beispielsweise 250 und 350 °C ist. Im Bereich der Einlaufzone weist die Doppelbandpresse 2 zwei übereinander angeordnete, in Lagerbrücken 3 bzw. 4 drehbar abgestützte Umlenktrommeln 5, 6 auf. Um jede Trommel 5, 6 ist ein endloses Pressband 7 bzw. 8 herum geführt. Die Pressbänder 7, 8 verlaufen ausserdem in an sich bekannter Weise über nicht dargestellte, weitere, ebenfalls an den Lagerbrücken 3, 4 abgestützte Umlenkrollen. Wie dargestellt (vgl. auch Fig. 2 und 3), besteht jedes Pressband 7, 8 aus einem Pressbandpaket, welches jeweils von vier ineinander geschobenen Bandtrumen 11, 12, 13, 14 bzw. 15, 16, 17, 18 gebildet wird. Die Bandtrume 14 bzw. 18 bilden jeweils die Aussenfläche des Pressbandpakets und greifen im Bereich der in Fig. 1 mit 10 bezeichneten Reaktionszone beidseitig an der Werkstoffbahn 1 an. Die Umlaufrichtung der Pressbänder ist durch Pfeile A angegeben.

Die in Fig. 1 von rechts nach links vorlaufende Werkstoffbahn 1 wird in der Reaktionszone 10 unter gleichzeitiger Anwendung von Wärme und Druck verdichtet. Die Umlenktrommeln 5, 6 sind in an sich bekannter Weise beheizt, so dass von ihnen Wärme auf die mehrlagigen Pressbänder 7, 8 übertragen wird.

Der auf die Werkstoffbahn 1 auszuübende Druck wird über Druckplatten 19, 20 in an sich bekannter Weise hydraulisch oder mechanisch auf das jeweils innen gelegene Bandtrum der Pressbänder 7, 8 aufgebracht und von dort über die einzelnen Bandlagen auf die Werkstoffbahn 1 übertragen. Bei der hydraulischen Druckübertragung wird ein unter Druck setzbares fluides Druckmedium in den Raum zwischen den Druckplatten 19, 20 und der Rückseite des innersten Bandtrums 11 bzw. 15 gebracht. Seitlich ist dieser Raum durch in der Zeichnung nicht dargestellte Dichtungen begrenzt, die in der Regel aus organischen, elastomeren Dichtungswerkstoffen mit nur begrenzter Wärmebeständigkeit bestehen. Bei der mechanischen Druckübertragung sind in den Zwischenraum zwischen den Druckplatten 19, 20 und der Rückseite der innersten Trume 11 bzw. 15 quer über die Pressbandbreite verlaufende Wälzkörper angeordnet. Die Druckplatten mit den Wälzkörpern werden über Druckmittelzylinder an das innen gelegene Bandtrum angedrückt. Die für die Wälzkörper benötigten Schmierstoffe besitzen bei erhöhter Temperatur ebenfalls eine geringe Zersetzungs- und Oxidationsstabilität.

In der Nähe des zwischen den Umlenktrommeln 5, 6 und den von ihnen abgestützten Pressbändern 7, 8 gebildeten Einlaufspalts 21, das heisst möglichst unmittelbar vor der Reaktionszone 10, erstrecken sich quer über die Breite der Pressbänder 7, 8 hinweg leistenförmige Gasbrenner 22, 23, deren Flammen unmittelbar auf die Aussenseite der aussen gelegenen Bandtrume 14 bzw. 18 einwirken. Durch die Wärme der Verbrennungsgase wird die Oberfläche dieser Bandtrume kurz vor dem Einlaufspalt 21 im Bereich einer «Flammzone» aufgeheizt, und zwar auf eine Temperatur, die höher als diejenige Temperatur liegt, auf welche die Pressbänder von den Umlenktrommeln 5, 6 her aufgeheizt werden. Anstelle der Gasbrenner 22, 23 können auch elektrische Induktionsheizkörper mit von hochfrequenten Wechselströmen durchflossenen Koppelspulen verwendet werden.

Bevor die umlaufenden Pressbänder 7, 8 die Gasbrenner 22, 23 erreichen, werden sie durch die beheizten Umlenktrommeln 5, 6 erwärmt. Wie aus der stark vergrösserten Darstellung der Fig. 2 hervorgeht, ist im Bereich der Einlaufzone 21 und davor ein Wärmekontakt zwischen den die Pressbänder 7, 8 bildenden Bandtrumen aufgrund von Oberflächenrauhigkeiten nur lokal gegeben, da die Pressbänder hier noch keinem Druck ausgesetzt sind. Somit ist der Wärmewiderstand an dieser Stelle gross und es existiert ein abnehmendes Wärmegefälle vom inneren Bandtrum 11 bzw. 15 zum äusseren Bandtrum 14 bzw. 18 hin. Die Temperatur der Umlenktrommeln 5, 6 kann so geregelt werden, dass auf dem inneren Bandtrum die für die Dichtungswerkstoffe, Schmiermittel oder dergleichen zulässige Betriebstemperatur, die sogenannte kritische Temperatur, nicht überschritten wird.

Das Temperaturgefälle in den Pressbandpaketen vor und hinter den von den Gasbrennern 22, 23 gebildeten «Flammzonen» ist schematisch in Fig. 3 dargestellt. Das Temperaturgefälle vor der Flammzone ist mit dem Bezugszeichen 24, dasjenige hinter der Flammzone mit dem Bezugszeichen 25 versehen. Die Temperatur der inneren Bandtrume 11, 15 liegt bei rund 200 °C, also unterhalb einer kritischen Temperatur von beispielsweise 240 °C. Die Höhe der betreffenden Temperatur ist jeweils durch entsprechende Schraffur am entsprechenden Bandtrum mit Bezug zu einer Temperaturskala angedeutet. Durch Wärmeleitung fliesst die Wärme von den innen gelegenen Bandtrumen 11, 15 zum äusseren Bandtrum 14 bzw. 18, wobei sich jedoch aufgrund des Wärmewiderstandes ein Wärmegefälle zu den äusseren Bandtrumen hin einstellt und die Temperatur dementsprechend dort bei rund 160 °C liegt.

In den im Bereich der Gasbrenner 22, 23 liegenden Flammzonen kehrt sich die Wärmeflussrichtung durch das starke Aufheizen der äusseren Bandtrume 14, 18 um. Dabei wird auf dem äusseren Bandtrum die durch die Brenner 22, 23 erzeugte Temperatur so gewählt, dass sie für die zu bearbeitende Werkstoffbahn 1 optimal ist. Dabei kann die vorerwähnte kritische Temperatur ohne weiteres überschritten werden, weil durch die schlechte Wärmeleitfähigkeit der lose aufeinander liegenden Bandtrume der Temperaturanstieg in den innersten Bandtrumen vernachlässigbar ist.

Im Bereich der Reaktionszone 10, wo der volle Druck über die Druckplatten 19, 20 auf die Pressbänder 7, 8 einwirkt, sinkt der Wärmewiderstand an den Übergängen zwischen den einzelnen Bandtrumen, da sich, wie in Fig. 2 dargestellt, die Wärmeübertragungsfläche unter der Druckeinwirkung vergrössert. Der Hauptanteil des Wärmeflusses findet jedoch von den durch die Brenner 22, 23 unmittelbar an ihren Aussenseiten zusätzlich aufgeheizten, aussen liegenden Bandtrumen 14 bzw. 18 auf die direkt anliegende Werkstoffbahn 1 statt und führt in dieser Bahn zu den dort erwünschten, höheren Temperaturen. Somit stellt sich das in Fig. 3 mit dem Bezugszeichen 25 bezeichnete Temperaturgefälle ein. Dabei haben die äusseren Bandtrume 14 bzw. 18, wie anhand der Schraffur dargstellt, eine weit oberhalb der kritischen Temperatur liegende Temperatur von etwa 350 °C. In den weiter innen gelegenen Bandtrumen der Pressbänder 7, 8 nimmt die Temperatur dagegen ständig ab und erreicht am innersten Bandtrum 11, 15 einen Wert, welcher die kritische Temperatur von etwa 240 °C nicht mehr übersteigt.

Die in Fig. 3 dargestellten Werte hängen von den jeweiligen Werkstoffen und den Betriebsparametern der Doppelbandpresse ab. Sie können an den jeweiligen Betriebsfall angepasst werden. Jedenfalls lässt sich in jedem Falle erreichen, dass im Bereich der Reaktionszone an der Aussenseite der äussersten Pressbandtrume die gewünschte hohe Prozesstemperatur erreicht ist, während an den Innenseiten der innen gelegenen Bandtrume die kritische Temperatur nicht überschritten wird.

Bei mehrlagigen Pressbändern verbessern sich die Wärmübergänge schlagartig, sobald die Bandpakete die Reaktionszone erreichen und unter hohen Flächendruck gelangen. Der damit einhergehende, an sich unerwünschte Wärmeübergang zu den Pressbandinnenseiten wird jedoch durch die beabsichtigte Wärmeaufnahme durch die Werkstoffbahn begrenzt. Ausserdem sorgen natürlich die bei mehrlagigen Pressbändern vorhandenen Grenzflächen dafür, dass sich der Wärmeübergang zu den empfindlichen Teilen und Werkstoffen auf der Maschineninnenseite hin in Grenzen hält.

Die durch die Erfindung erzielbaren Vorteile sind vor allem wirtschaftlicher Natur. Die Werkstoffbahnen werden erfindungsgemäss kontinuierlich verpresst und können rollenförmig aufgewickelt werden. Hierdurch ergeben sich gegenüber bogenförmigen Werkstoffen, wie sie zum Beispiel mit Hilfe von Ein- oder Mehretagenpressen herstellbar sind, je nach Zuschnittgrösse bis zu 15% höhere Ausbeuten. Da hochtemperaturbeständige Werkstoffbahnen in Form von Laminaten oder Faser-Bindemittelgemischen regelmässig sehr teure Rohstoffe erfordern, kann die höhere Ausbeute die Kosten der Verarbeitungsvorrichtung in kürzester Zeit kompensieren.

Manche Faser-Bindemittelgemische sind nahezu ideale Wärmeisolatoren, die bisher nur durch konvektive Wärmeübertragung auf die erforderliche Verdichtungstemperatur gebracht werden konnten. Hierfür eignen sich zwar die bekannten Etagenpressen, ihre Heizplatten erfordern jedoch bis zu zwei Grössenordnungen höhere Wärmekapazitäten, die bei erforderlicher Rückkühlung und hohen Verdichtungstemperaturen einen entsprechend hohen Energieverbrauch mit sich bringen. Auch hier schafft die Erfindung Abhilfe.

**Patentansprüche**

1. Verfahren zum kontinuierlichen Verpressen von Werkstoffbahnen (1) in einer Reaktionszone (10) zwischen zwei endlosen, aufgeheizten Pressbändern (7, 8) einer Doppelbandpresse (2) bei erhöhter Prozesstemperatur, wobei die Pressbänder (7, 8) so aufgeheizt werden, dass sie an ihren der Werkstoffbahn (1) zugekehrten Aussenseiten die höchste Temperatur haben und der Wärmefluss durch das Pressband (7, 8) von dieser heissen Aussenseite zur gegenüberliegenden Innenseite des Pressbandes (7, 8) hin erfolgt, dadurch gekennzeichnet, dass das Pressband (7, 8) aus mehreren, ineinander geschobenen Bandtrumen (11, 12, 13, 14 bzw. 15, 16, 17, 18) besteht, alle Bandtrume (11, 12, 13, 14 bzw. 15, 16, 17, 18) vor der Reaktionszone (10) von ihrer Innenseite her vorgeheizt werden und lediglich die Aussenseite des aussen gelegenen Bandtrums (14 bzw. 18) kurz vor der Reaktionszone (10) zusätzlich auf erhöhte Temperatur gebracht wird, so dass der Wärmefluss von der heissen Aussenseite des aussenliegenden Bandtrums (14 bzw. 18) zur gegenüberliegenden Innenseite des innenliegenden Bandtrums (11 bzw. 15) mit hohem Wärmewiderstand erfolgt.

2. Vorrichtung zur Durchführung des Verfahrens nach Anspruch 1 mit einer Doppelbandpresse (2), die zwei übereinander angeordnete, über Umlenktrommeln (5, 6) geführte endlose Pressbänder (7, 8) besitzt, wobei die der Einlaufzone in die Doppelbandpresse (2) zugeordneten Umlenktrommeln (5, 6) beheizbar sind und mit Einrichtungen zur Erwärmung der der Werkstoffbahn (1) zugekehrten Aussenseiten der Pressbänder (7, 8), dadurch gekennzeichnet, dass in der Nähe der Aussenseite des aussenliegenden Bandtrums (14, 18) des aus mehreren, ineinander geschobenen Bandtrumen (11, 12, 13, 14 bzw. 15, 16, 17, 18) bestehenden Pressbandes, (7, 8) kurz vor der Reaktionszone (10) quer über die Breite der Pressbänder (7, 8) reichende, leistenförmige Gasbrenner (22, 23) angeordnet sind.

3. Vorrichtung zur Durchführung des Verfahrens nach Anspruch 1 mit einer Doppelbandpresse (2), die zwei übereinander angeordnete, über Umlenktrommeln (5, 6) geführte endlose Pressbänder (7, 8) besitzt, wobei die der Einlaufzone in die Doppelbandpresse (2) zugeordneten Umlenktrommeln (5, 6) beheizbar sind und mit Einrichtungen zur Erwärmung der der Werkstoffbahn (1) zugekehrten Aussenseiten der Pressbänder (7, 8), dadurch gekennzeichnet, dass in der Nähe der Aussenseiten des aussenliegenden Bandtrums (14, 18) des aus mehreren, ineinander geschobenen Bandtrumen (11, 12, 13, 14 bzw. 15, 16, 17, 18) bestehenden Pressbandes (7, 8), kurz vor der Reaktionszone (10) quer über die Breite der Pressbänder (7, 8) reichende, elektrische Induktionsheizkörper mit von hochfrequenten Wechselströmen durchflossenen Koppelspulen angeordnet sind.

## Claims

1. Method for the continuous pressing of materials webs (1) in a reaction zone (10) between two endless heated press bands (7, 8) of a double band press (2) at elevated process temperature, wherein the press bands (7, 8) are so heated that they have the highest temperature at their outer sides facing the material web (1) and the heat flow through the press band (7, 8) takes place from this hot outer side towards the oppositely disposed inner side of the press band (7, 8), characterised thereby, that the press band (7, 8) consists of several band runs (11, 12, 13, 14 or 15, 16, 17, 18) pushed one inside the other, all band runs (11, 12, 13, 14 or 15, 16, 17, 18) are preheated from their inner side before the reaction zone (10) and merely the outer side of the outwardly disposed belt run (14 or 18) is additionally brought to elevated temperature shortly before the reaction zone (10) so that the heat flow from the hot outer side of the outwardly disposed belt run (14 or 18) towards the oppositely disposed inner side of the inwardly disposed belt run (11 or 15) takes place with high thermal resistance.

2. Apparatus for the performance of the method according to claim 1 with a double band press (2), which possesses two endless press bands (7, 8), which are arranged one above the other and guided over deflecting drums (5, 6), wherein the deflecting drums (5, 6) associated with the inlet zone into the double band press (2) are heatable, and with equipments for heating those outer sides of the press bands (7, 8), which face the material web (1), characterised thereby, that strip-shaped gas burners (22, 23), which reach transversely over the width of the press bands (7, 8), are arranged shortly before the reaction zone (10) in the proximity of the outer sides of the outwardly disposed belt run (14 or 18) of the press band (7, 8) consisting of several band runs (11, 12, 13, 14 or 15, 16, 17, 18) pushed one inside the other.

3. Apparatus for the performance of the method according to claim 1 with a double band press (2), which possesses two endless press bands (7, 8), which are arranged one above the other and guided over deflecting drums (5, 6), wherein the deflecting drums (5, 6) associated with the inlet zone into the double band press (2) are heatable, and with equipments for heating those outer sides of the press bands (7, 8), which face the material web (1), characterised thereby, that electrical induction heater elements, which reach transversely over the width of the press bands (7, 8), with coupling coils flowed through by high-frequency alternating currents, are arranged shortly before the reaction zone (10) in the proximity of the outer sides of the outwardly disposed belt run (14 or 18) of the press band (7, 8) consisting of several band runs (11, 12, 13, 14 or 15, 16, 17, 18) pushed one inside the other.

## Revendications

1. Procédé pour comprime d'une manière continue des bandes (1) de matériaux dans une zone de réaction (10) comprise entre deux bandes de pressage (7, 8) sans fin, chauffées, d'une double presse à bandes (2), sous une température d'opération accrue, les bandes de pressage (7, 8) étant chauffées de telle sorte, qu'elles présentent la température la plus élevée sur leurs faces extérieures, situées en regard de la bande de matériau (1) et que le flux de chaleur à travers la bande de pressage (7, 8) s'effectue en passant de cette face extérieure chaude à la face intérieure, située à l'opposé, de la bande de pressage (7, 8), procédé caractérisé en ce que la bande de pressage (7, 8) est constituée par plusieurs couches de bandes (11, 12, 13, 14 ou 15, 16, 17, 18) serrées les unes dans les autres, toutes les couches de bandes (11, 12, 13, 14 et 15, 16, 17, 18) sont préchauffées en avant de la zone de réaction (10) depuis leur face intérieure, et uniquement la face extérieure de la couche de bande (14 ou 18) située extérieurement est portée en plus, juste en avant de la zone de réaction (10), à une température accrue de telle sorte que le flux de chaleur s'effectue en passant de la face extérieure chaude de la couche de bande (14 ou 18), située extérieurement, à la face intérieure, située à l'opposé, de la couche de bande (11 ou 15) située intérieurement, à haute résistance thermique.

2. Dispositif pour la mise en oeuvre du procédé selon la revendication 1, comportant une double presse à bandes (2), qui est pourvue de deux bandes de pressage (7, 8) sans fin, disposées l'une au-dessus de l'autre et passant sur des tambours de renvoi (5, 6), les tambours de renvoi (5, 6) associés à la zone d'entrée dans la double presse à bandes (2) pouvant être chauffées, le dispositif comportant également des appareils pour réchauffer les faces extérieures des bandes de pressage (7, 8) situées en regard de la bande de matériau (1), dispositif caractérisé en ce que des brûleurs à gaz (22, 23) en forme de barres sont montés juste en avant de la zone de réaction (10) en s'étendant transversalement sur la largeur des bandes de pressage (7, 8), à proximité des faces extérieures de la couche de bande (14, 18), située extérieurement, de la bande de pressage (7, 8), située extérieurement, de la bande de pressage (7, 8) constituée par plusieurs couches de bandes (11, 12, 13, 14 ou 15, 16, 17, 18) serrées les unes dans les autres.

3. Dispositif pour la mise en oeuvre du procédé selon la revendication 1, comportant une double presse à bandes (2), qui est pourvue de deux bandes de pressage (7, 8) sans fin, disposées l'une au-dessus de l'autre et passant sur des tambours de renvoi (5, 6), les tambours de renvoi (5, 6) associés à la zone d'entrée dans la double presse à bandes (2) pouvant être chauffés, et le dispositif comportant également des appareils pour réchauffer les faces extérieures des bandes de pressage (7, 8) situées en regard de la bande de matériau (1), dispositif caractérisé en ce que des éléments chauffants électriques à induction pourvus de bobines de couplage parcourues par des courants alternatifs à haute fréquence sont montés juste en avant de la zone de réaction (10) en s'étendant transversalement sur la largeur des bandes de pressage (7, 8), à proximité des faces extérieures de la couche de bande (14, 18), située extérieurement, de la bande de pressage (7, 8) constituée par plusieurs couches de bandes (11, 12, 13, 14 ou 15, 16, 17, 18) serrées les unes dans les autres.

# Fig.1

# Fig.2

# Fig.3